# EUROPEAN PATENT SPECIFICATION

(11) **EP 3 937 126 B1**
(45) Date of publication and mention of the grant of the patent: **16.04.2025**
(21) Application number: 20184995.7
(22) Date of filing: 09.07.2020
(51) Int. Cl.: G06T 7/00, G16H 30/00, A61B 1/00

(54) **ENDOSCOPE IMAGE PROCESSING DEVICE**
VERARBEITUNGSVORRICHTUNG FÜR ENDOSKOPBILDER
DISPOSITIF DE TRAITEMENT D'IMAGES D'ENDOSCOPE

(43) Date of publication of application: 12.01.2022
(73) Proprietor: Ambu A/S, 2750 Ballerup (DK)
(72) Inventor: JØRGENSEN, Andreas Härstedt, 2610 Rødovre (DK); SONNENBORG, Finn, 3600 Frederikssund (DK)
(74) Representative: AWA Denmark A/S

(56) References cited:
- WO-A1-2018/195216
- US-A1- 2016 309 992
- US-A1- 2018 296 281
- US-A1- 2020 054 400

## Description

### Field

The present disclosure relates to an image processing device for estimating a landing zone of a medical device extendable from a tip of an endoscope, a display unit comprising an image processing device, an endoscope system comprising an endoscope and an image processing device, and a method of performing a medical procedure.

### Background

Endoscopes are widely used in hospitals for visually examining body cavities and obtaining samples of tissue identified as potentially pathological. An endoscope typically comprises an image capturing unit arranged at the distal end of the endoscope either looking forward or to the side. An endoscope is further typically provided with a working channel allowing a medical device such as a gripping device, a suction device, or a catheter to be introduced.

It may however be difficult for a number of medical procedures to secure that that the medical device reaches its intended destination. Endoscopic retrograde cholangiopancreatography, ERCP, is an example of such a medical procedure. In ERCP the major duodenal papilla is catheterized using a catheter advanced from the tip of duodenoscope. The duodenoscope is provided with a guiding element is the form of an elevator element for guiding the catheter in a particular direction. By controlling the elevator element and positioning the duodenoscope correctly the catheter may be introduced into the major duodenal papilla. This may however by a challenging and time-consuming process.

US2020054400 discloses an image processing apparatus including a processor including hardware, the processor being configured to: estimate, based on image information from a medical device that includes at least an endoscope, a plurality of procedure actions of an operator of the endoscope, perform different supports respectively for procedures by the operator, according to an estimation result of the procedure actions; and output a display for the supports to a display.

Thus it remains a problem to provide an improved device / system for performing endoscopic procedures.

### Summary

According to a first aspect, the present disclosure relates to an image processing device for estimating a landing zone of a medical device extendable from a tip of an endoscope, the endoscope comprising one or more sensors including an image capturing device, the image processing device comprising a processing unit operationally connectable to the one or more sensors of the endoscope, wherein the processing unit is configured to:
obtain a stream of images captured by the image capturing device of the endoscope;
process first sensor data recorded by the one or more sensors to estimate the landing zone of the medical device; and
provide the stream of images with a visual indication, indicating the estimated landing zone.

Consequently, the operator of the endoscope may directly be provided with visual aid for assisting with performing a successful procedure.

The image processing device may be built into a display unit or it may be a standalone device operationally connectable to both the one or more sensors of the endoscope and a display unit. The image capturing device may be the only sensor of the endoscope, i.e. the one or more sensors may consist of the image capturing device. The landing zone may be the position the medical device will reach when extended a predetermined length from the endoscope.

The processing unit of the image processing device may be any processing unit, such as a central processing unit (CPU), a graphics processing unit (GPU), a microcontroller unit (MCU), a field-programmable gate array (FPGA), or any combination thereof. The processing unit may comprise one or more physical processors and/or may be combined by a plurality of individual processing units.

In some embodiments, the processing unit is further configured to obtain an identification of the type of medical instrument, and wherein the estimated landing zone is further dependent on the identification of the type of medical instrument.

The processing unit may obtain the identification via user input and / or automated e.g. by processing sensor data from the one or more sensors.

The visual indication may be a visual element overlaid on the stream of images.

The visual element may comprise parts that are either fully or partly transparent. Thus, visual content of the stream of images may only to a small degree be covered by the visual element.

The visual element may be arranged at the estimated landing zone. Alternatively, the visual element may be arranged next to the estimated landing zone. Arranging the visual element next to the estimated landing zone may be beneficial as the landing zone typically is at a point of interest that optimally should be freely visible to the user.

In some embodiments, the processing unit is further configured to estimate a part of the trajectory by processing the first sensor data, and wherein the visual indication further indicates the estimated trajectory.

By further estimating and indicating the trajectory of the medical device procedures requiring a precise orientation between the medical device and the point of interest may be performed easier and safer. ERCP, is an example of such a medical procedure. In ERCP the major duodenal papilla is catheterized using a catheter advanced from the tip of duodenoscope where the orientation between the catheter and the major duodenal papilla is crucial for the success of the procedure. Removal of polyps may also require a precise orientation between the polyp and a cutting tool.

In some embodiments, the processing unit is further configured to:
obtain an identification of an anatomic landmark in the stream of images;
determine the location of the anatomic landmark in the stream of images;
determine if the estimated landing zone intersects with the determined location of the anatomic landmark.

Consequently, a user may be provided with further assistance.

In some embodiments, the processing unit is further configured to:
obtain the identification of the anatomic landmark by processing on or more images of the stream of images.

Consequently, the system may be at least partly automated.

In some embodiments, the processing unit is configured to obtain the identification of the anatomic landmark by processing the stream of images using a machine learning data architecture trained to identify the anatomic landmark in endoscope images.

The machine learning data architecture may be a supervised machine learning architecture, trained by being provided with a training data set of images from a large number of patients, where a first subset of images of the training data set includes the anatomic landmark and a second subset of images does not include the anatomic landmark.

In some embodiments, the machine learning data architecture is an artificial neural network such as a deep structured learning architecture.

Additionally / alternatively, in some embodiments, the processing unit is further configured to:
obtain the identification of the anatomic landmark through a user input.

As an example the processing unit may be operationally connected to a touch screen displaying the stream of images live to the user, where the user may manually identify the anatomic landmark e.g. by clicking on it on the touchscreen. The processing unit may then use motion tacking techniques in order to keep track of the anatomic landmark as the endoscope moves around.

In some embodiments, the processing unit is further configured to:
provide the stream of images with a visual indication, indicating that the estimated landing zone intersects with the determined location of the anatomic landmark.

Consequently, the user may be provided with further assistance when performing a medical procedure. This may make the medical procedure easier and safer.

In some embodiments, the first sensor data is one or more images of the stream of images.

Consequently, by utilizing one or more images from the stream of images for estimating the landing zone, the endoscope does not need be provided with further sensors.

In some embodiments, the one or more images of the stream of images are processed using a machine learning data architecture trained to estimate the landing zone of the medical device.

The machine learning data architecture may be a supervised machine learning architecture.

In some embodiments, the machine learning data architecture is an artificial neural network such as a deep structured learning architecture.

In some embodiments, the endoscope further comprises a guiding element configured to guide the medical device in a particular direction, and wherein the one or more sensors further comprises a guiding element sensor for detecting the position and / or orientation of the guiding element, and wherein the first sensor data is recorded by the guiding element sensor.

In some embodiments, the endoscope is duodenoscope and wherein the guiding element is an elevator element configured to elevate a catheter.

According to a second aspect the disclosure relates to a display unit for displaying images obtained by an image capturing device of an endoscope, wherein the display unit comprises an image processing device as disclosed in relation to the first aspect of the disclosure.

According to a third aspect the disclosure relates to an endoscope system comprising an endoscope and an image processing device as disclosed in relation to the first aspect, wherein the endoscope has an image capturing device and the processing unit of the image processing device is operationally connectable to the image capturing device of the endoscope.

The endoscope may comprise a working channel for allowing a medical device to extend from its tip. The endoscope may further comprise a guiding element for guiding the medical device in a particular direction. The guiding element may be movable from a first position to a second position. The guiding element may be provided with or without a guiding element sensor. The guiding element may be controllable from the handle of the endoscope. The guiding element may be configured guide the medical device in a particular direction relative to the tip of the endoscope. Thus, dependent on the position of the guiding element the landing zone of the medical device will be arranged at the different parts of the endoscope image.

The endoscope may be a duodenoscope and wherein the guiding element is an elevator element configured to elevate a catheter.

According to a fourth aspect the disclosure relates to a method of training a machine learning data architecture for estimating a landing zone of a medical device extendable from a tip of an endoscope, comprising the steps of:
providing a training data set comprising a plurality of images captured by an image capturing device of an endoscope,
providing for each image the position of the resulting landing zone of the medical device.

In some embodiments, the endoscope comprises a guiding element for guiding the medical device in a particular direction, wherein the guiding element is movable from a first position to a second position, and wherein the guiding element is arranged in different positions in the plurality of images.

In some embodiments, a part of the medical device can be seen in at least some of the plurality of images.

In some embodiments, the plurality of images includes images of different medical devices.

According to a fifth aspect the disclosure relates to a method of performing a medical procedure using an endoscope system as disclosed in relation to the third aspect of the disclosure, comprising the steps of:
advancing the endoscope through a body to a position near a point of interest,
extending a medical device from the endoscope to a treatment position based on a stream of visual images provided with a visual indication, indicating an estimated landing zone of the medical device,
performing a medical procedure at the treatment position, such as removing a polyp, catherizing the major duodenal papilla or cutting a larger opening in the major duodenal papilla.

The different aspects of the present disclosure can be implemented in different ways including image processing devices, display units, endoscope systems, methods of training a machine learning data architecture, and methods of performing a medical procedure described above and in the following, each yielding one or more of the benefits and advantages described in connection with at least one of the aspects described above, and each having one or more preferred embodiments corresponding to the preferred embodiments described in connection with at least one of the aspects described above and/or disclosed in the dependant claims. Furthermore, it will be appreciated that embodiments described in connection with one of the aspects described herein may equally be applied to the other aspects.

### Brief description of the drawings

The above and/or additional objects, features and advantages of the present disclosure, will be further elucidated by the following illustrative and non-limiting detailed description of embodiments of the present disclosure, with reference to the appended drawings, wherein:
Fig. 1 shows an example of an endoscope.
Fig. 2 shows an example of a display unit that can be connected to the endoscope shown in Fig. 1.
Fig. 3 shows a schematic drawing of an endoscope system according to an embodiment of the disclosure.
Fig. 4 shows a schematically drawing of an endoscope system according to an embodiment of the disclosure.
Fig 5a-d show schematically images captured by an image capturing device of an endoscope according to an embodiment of the disclosure.
Figs 6a-b show images captured by an image capturing device of an endoscope according to an embodiment of the disclosure
Fig. 7 shows a flowchart of a method of training a machine learning data architecture for estimating a landing zone of a medical device extendable from a tip of an endoscope according to an embodiment of the disclosure.
Fig. 8 shows a flowchart of a method of performing a medical procedure using an endoscope system according to an embodiment of the

### disclosure. Detailed description

In the following description, reference is made to the accompanying figures, which show by way of illustration how the embodiments of the present disclosure may be practiced.

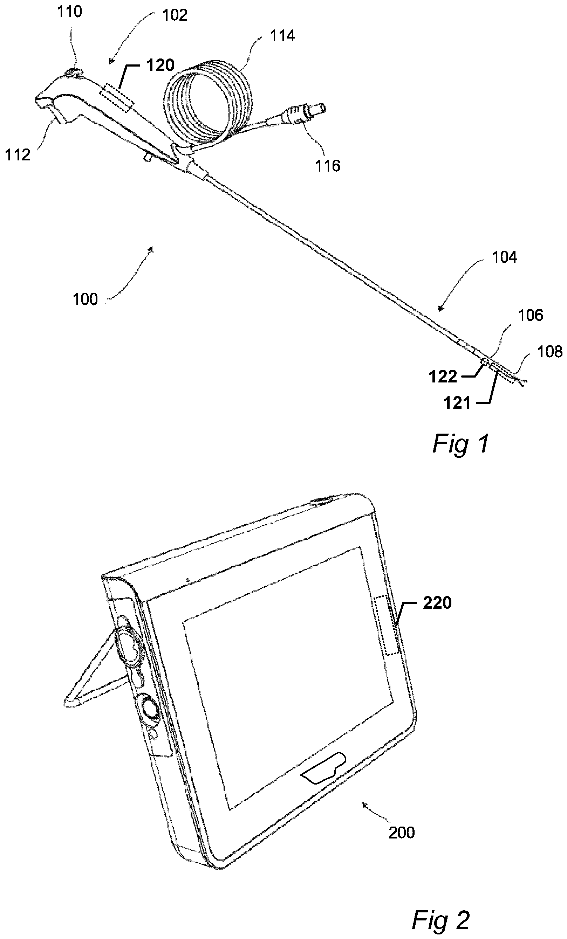

Fig. 1 illustrates an example of an endoscope 100. This endoscope may be adapted for single-use. The endoscope 100 is provided with a handle 102 attached to an insertion tube 104 provided with a bending section 106. The insertion tube 104 as well as the bending section 106 may be provided with one or several working channels such that instruments, such as a gripping device or a catheter, may extend from the tip and be inserted into a human body via the endoscope. One or several exit holes of the one or several channels may be provided in a tip part 108 of the endoscope 100. In addition to the exit holes, a camera sensor, such as a CMOS sensor or any other image capturing device, as well as one or several light sources, such as light emitting diodes (LEDs), fiber, or any other light emitting devices, may be placed in the tip part 108. By having the camera sensor and the light sources and a monitor 200, illustrated in Fig. 2, configured to display images based on image data captured by the camera sensor, an operator is able to see and analyze an inside of the human body in order to for instance localize a position for taking a sample. In addition, the operator will be able to control the instrument in a precise manner due to the provided visual feedback. Further, since some diseases or health issues may result in a shift in natural colors or other visual symptoms, the operator is provided with valuable input for making a diagnosis based on the image data provided via the image capturing device and the monitor.

The endoscope may further comprise a guiding element 121 for guiding the medical device in a particular direction (only schematically shown). The guiding element 121 may be movable from a first position to a second position. The guiding element may be controllable from the handle of the endoscope via an actuation element 120 (only schematically shown). The guiding element may be configured guide the medical device in a particular direction relative to the tip of the endoscope. Thus, dependent on the position of the guiding element 121 the landing zone of the medical device will be arranged at the different parts of the endoscope image.

The endoscope may further optionally comprise a guiding element sensor 122. The guiding element sensor 122 may detect the position of the guiding element, which may then be used to estimate the landing zone a medical device extendable from the tip of the catheter. However, in some embodiment the endoscope does not comprise a guiding element sensor 122, and the landing zone of the medical device is estimated only using image data recorded by the image capturing device.

In order to make it possible for the operator to direct the camera sensor such that different field of views can be achieved, the endoscope has a bending section 106 that can be bent in different directions with respect to the insertion tube 104. The bending section 106 may be controlled by the operator by using a knob 110 placed on the handle 102. The handle 102 illustrated in fig 1 is designed such that the knob 110 is controlled by a thumb of the operator, but other designs are also possible. In order to control a gripping device or other device provided via a working channel a push button 112 may be used. The handle 102 illustrated in fig 1 is designed such that a index finger of the operator is used for controlling the gripping device, but other designs are also possible.

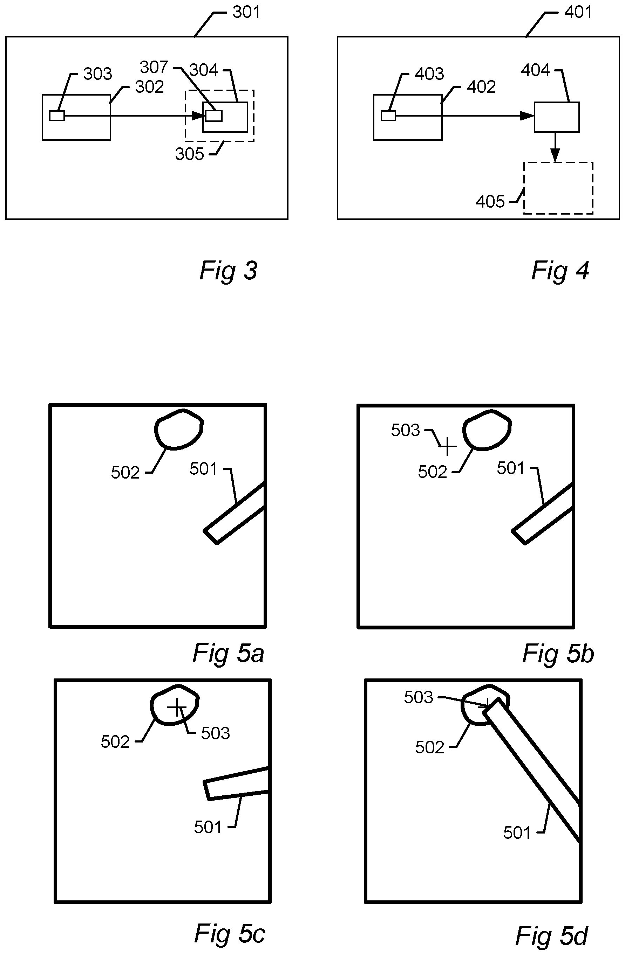

Fig. 3 shows a schematic drawing of an endoscope system 301 according to an embodiment of the disclosure. The endoscope system 301 comprises an endoscope 302 and an image processing device 304. The endoscope 302 has a working channel allowing a medical instrument to extend from the tip of the endoscope 302. The image processing device comprises a processing unit 307. The endoscope 302 comprises one or more sensors including an image capturing device 303. The processing unit 307 of the image processing device 304 is operationally connectable to the image capturing device of the endoscope 303. The processing unit 307 is configured to:
obtain a stream of images captured by the image capturing device of the endoscope; process first sensor data recorded by the one or more sensors to estimate the landing zone of the medical device; and provide the stream of images with a visual indication, indicating the estimated landing zone. In this embodiment, the image processing device 304 is integrated in a display unit 305. Consequently, the user may in real time be assisted when performing complex medical procedures such as catherization of the major duodenal papilla. The landing zone may be the position the medical device will reach when extended a predetermined length from the endoscope.

The position of the landing zone may dependent on the type of medical instrument and / or the orientation of the medical instrument. As an example if the medical instrument e.g. a catheter, is provided with a tip having a prebend then the landing zone will dependent of the orientation of the medical instrument within the working channel. The one or more sensors of the endoscope may include a sensor configured to detect the type of medical instrument in the working channel and / or the orientation of the medical instrument. However, the processing may also be configured to estimate the landing zone by analyzing one or more images of the stream of images e.g. one or more images showing the type of medical instrument and / or the orientation of the medical instrument e.g. after the medical instrument has propagated a short distance out of the working channel.

The one or more images of the stream of images may be processed using a machine learning data architecture trained to estimate the landing zone of the medical device. The machine learning data architecture may be trained by being provided with a training data set comprising a larger number of images showing medical instruments extended a short distance from the tip of the endoscope and further data of the resulting landing zones.

Additionally / alternatively, the position of the landing zone may be dependent on the position of a guiding element configured to guide the medical device in a particular direction. As an example, if the endoscope is duodenoscope the guiding element is an elevator element configured to elevate a catheter enabling the catheter to catherize the major duodenal papilla. The one or more sensors of the endoscope may include a guiding element sensor configured to detect the position and / or orientation of the guiding element.

However, the processing may also be configured to estimate the landing zone by analyzing one or more images of the stream of images e.g. one or more images showing the medical instrument and / or the guiding element e.g. after the medical instrument has propagated a short distance out of the working channel.

The one or more images of the stream of images may be processed using a machine learning data architecture trained to estimate the landing zone of the medical device. The machine learning data architecture may be trained by being provided with a training data set comprising a larger number of images showing medical instruments extended a short distance from the tip of the endoscope where the guiding element is positioned and / or oriented in different positions / orientations and further data of the resulting landing zones. If the position / orientation of the guiding element is visible to the image capturing device, then the images of the training data set may not include a medical instrument.

Fig. 5a-5d show schematically images captured by an image capturing device of an endoscope according to an embodiment of the disclosure. Fig. 5a shows a traditional unaltered image captured by an image capturing device of an endoscope. In the image a medical device 501 e.g. a cutting tool or a catheter, and an area of interest 502 can be seen. The area of interest may be polyp that needs to be removed using a cutting tool or an opening that needs to be catherized.

Fig. 5b shows an image captured by an image capturing device of an endoscope provided with a visual indication 501, indicating the estimated landing zone of the medical device 501 e.g. the position the medical device 501 will reach when extended a predetermined length from the endoscope. In this embodiment the visual indication 503 marks the center of the landing zone. Thus, since the visual indication 503 it not positioned at the area of interest 502 the user will have to re-arranged the endoscope in order to secure that the landing zone will be positioned at the area of interest 502. This may be done by moving the entire endoscope. Additionally / alternatively if the endoscope comprises a guiding element configured to guide the medical device 501 in a particular direction the user may move the guiding element so that the medical device is guided in a different direction.

Fig. 5c shows an image captured by an image capturing device of an endoscope provided with a visual indication 503, indicating the estimated landing zone of the medical device 501 after a guiding element of the endoscope has been moved. As a result, the medical device 501 is now arranged with a different angle in the image and the visual indication 503 now has moved and is positioned at the area of interest 502. The user can now safely extend the medical device 501 to the area of interest 502.

Fig. 5d shows an image captured by an image capturing device of an endoscope provided with a visual indication 503 indicating the estimated landing zone of the medical device 501. In this image the medical device 501 has been extended a distance and has reached the estimated landing zone 503. By providing the images with a visual indication 503 indicating the estimated landing zone procedures may be conducted faster and safer.

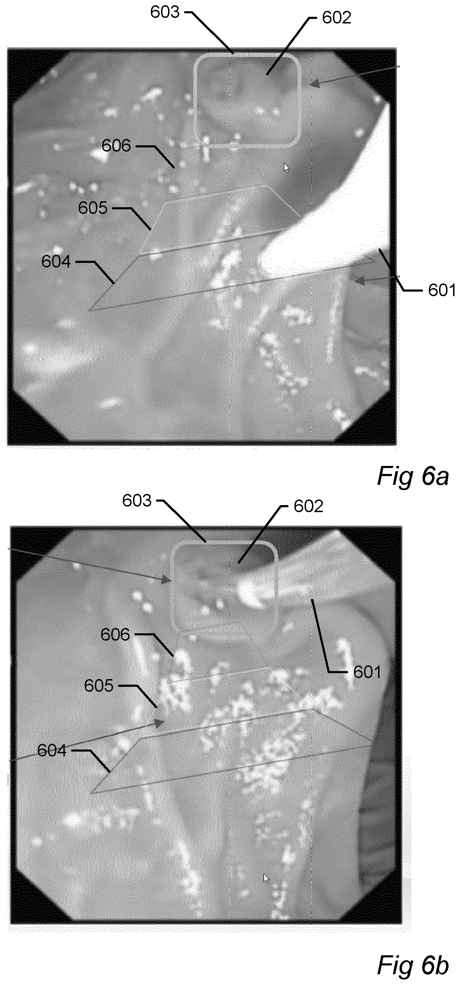

Figs 6a-b show images captured by an image capturing device of an endoscope according to an embodiment of the disclosure. In this embodiment the endoscope is a duodenoscope and the medical doctor is catheterizing the major duodenal papilla as part of an ERCP procedure.

In Fig. 6a a catheter 601 and the major duodenal papilla 602 is shown. The catheter 601 extends a distance from the duodenoscope. The image has further been provided with a visual indication 604 605 606 indicating an estimated trajectory and landing zone of the catheter 601. The image has further been provided with a visual indication 603 showing the location of the major duodenal papilla 602 in the image. In Fig. 6a the visual indication 604 605 606 indicating the estimated trajectory and landing zone of the catheter does is not aligned with the major duodenal papilla 602. This may be signalled to the user by displaying the visual indication 603 with a first colour e.g. a yellow colour. If the user then move the duodenoscope and or an elevator element of the duodenoscope so that the estimated trajectory and landing zone of the catheter is aligned with the major duodenal papilla 602 then the visual indication 603 may be displayed with a second colour e.g. a green colour.

Fig. 6b shows an image after the major duodenal papilla 602 has been catheterized with the catheter 601.

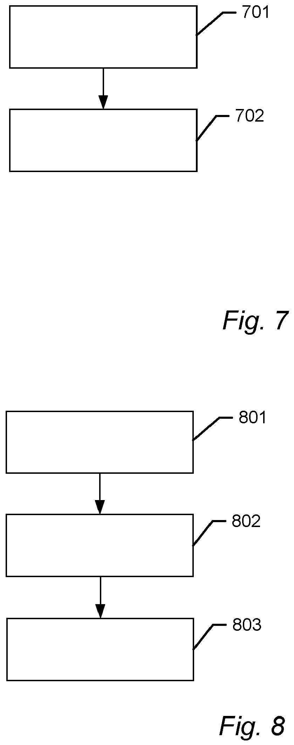

Fig. 7 shows a flowchart of a method of training a machine learning data architecture for estimating a landing zone of a medical device extendable from a tip of an endoscope according to an embodiment of the disclosure. In step 701 a training data set comprising a plurality of images captured by an image capturing device of an endoscope is provided. The images may show a part of the medical device. Next, in step 702 for each image the position of the resulting landing zone of the medical device is provided.

Fig. 8 shows a flowchart of a method of performing a medical procedure using an endoscope system according to an embodiment of the disclosure. In step 801 the endoscope is advanced through a body to a position near a point of interest. Then in step 802, a medical device is extended from the endoscope to a treatment position based on a stream of visual images provided with a visual indication, indicating an estimated landing zone of the medical device. Finally, in step 803 a medical procedure is performed at the treatment position, such as removing a polyp, catherizing the major duodenal papilla or cutting a larger opening in the major duodenal papilla.

## Claims

1. An image processing device (304) for estimating a landing zone of a medical device (501, 601) extendable from a tip of an endoscope (302), the landing zone being the position the medical device will reach when extended a predetermined length from the endoscope (302), the endoscope (302) comprising one or more sensors including an image capturing device (303), the image processing device (304) comprising a processing unit (307) operationally connectable to the one or more sensors of the endoscope (302), wherein the processing unit (307) is configured to:
obtain a stream of images captured by the image capturing device (303) of the endoscope (302);
process first sensor data recorded by the one or more sensors to estimate the landing zone of the medical device (501, 601); and
provide the stream of images with a visual indication (503, 603), indicating the estimated landing zone;
wherein the first sensor data is one or more images of the stream of images; and
wherein the one or more images of the stream of images are processed using a machine learning data architecture trained to estimate the landing zone of the medical device (501,601).

2. An image processing device according to claim 1, wherein the processing unit is further configured to estimate a part of the trajectory of the medical device as it extends from the endoscope, by processing the first sensor data, and wherein the visual indication further indicates the estimated trajectory.

3. An image processing device according to claims 1 or 2, wherein the processing unit is further configured to:
obtain an identification of an anatomic landmark in the stream of images;
determine the location of the anatomic landmark in the stream of images;
determine if the estimated landing zone intersects with the determined location of the anatomic landmark.

4. An image processing device according to claim 3, wherein the processing unit is further configured to:
obtain the identification of the anatomic landmark by processing one or more images of the stream of images.

5. An image processing device according to claim 4, wherein the processing unit is configured to obtain the identification of the anatomic landmark by processing the stream of images using a machine learning data architecture trained to identify the anatomic landmark in endoscope images.

6. An image processing device according to claim 3, wherein the processing unit is further configured to:
obtain the identification of the anatomic landmark through a user input.

7. An image processing device according to any one of claims 3 to 6, wherein the processing unit is further configured to:
provide the stream of images with a visual indication, indicating that the estimated landing zone intersects with the determined location of the anatomic landmark.

8. An image processing device according to any one of claims 1 to 7, wherein the endoscope further comprises a guiding element configured to guide the medical device in a particular direction, and wherein the one or more sensors further comprises a guiding element sensor for detecting the position and / or orientation of the guiding element, and wherein the first sensor data includes data recorded by the guiding element sensor.

9. A display unit for displaying images obtained by an image capturing device of an endoscope, wherein the display unit comprises an image processing device according to any one of claims 1 to8.

10. An endoscope system comprising an endoscope and an image processing device according to any one of claims 1 to 8, wherein the endoscope has an image capturing device and the processing unit of the image processing device is operationally connectable to the image capturing device of the endoscope.

11. An endoscope system according to claim 10, wherein the endoscope has a guiding element configured to guide the medical device in a particular direction and wherein the endoscope is a duodenoscope and wherein the guiding element is an elevator element configured to elevate a catheter.

12. A method of training a machine learning data architecture for estimating a landing zone of a medical device (501, 601) extendable from a tip of an endoscope (302), the landing zone being the position the medical device (501, 601) will reach when extended a predetermined length from the endoscope (302), comprising the steps of:
providing a training data set comprising a plurality of images captured by an image capturing device (303) of an endoscope (302),
providing for each image the position of the resulting landing zone of the medical device (501, 601).

13. A method according to claim12, wherein the endoscope comprises a guiding element for guiding the medical device in a particular direction, wherein the guiding element is movable from a first position to a second position, and wherein the guiding element is arranged in different positions in the plurality of images.

## Patentansprüche

1. Bildverarbeitungsvorrichtung (304) zum Schätzen einer Landezone einer medizinischen Vorrichtung (501, 601), die aus einer Spitze eines Endoskops (302) ausfahrbar ist, wobei die Landezone die Position ist, die die medizinische Vorrichtung erreichen wird, wenn eine vorbestimmte Länge aus dem Endoskop (302) ausgefahren ist, wobei das Endoskop (302) einen oder mehrere Sensoren inklusive einer Bildaufnahmevorrichtung (303) umfasst, wobei die Bildverarbeitungsvorrichtung (304) eine Verarbeitungseinheit (307) umfasst, die funktionell mit dem einen oder den mehreren Sensoren des Endoskops (302) verbunden werden kann, wobei die Verarbeitungseinheit (307) dazu konfiguriert ist:
einen Strom von Bildern zu erhalten, die von der Bildaufnahmevorrichtung (303) des Endoskops (302) aufgenommen werden;
erste Sensordaten zu verarbeiten, die von dem einen oder den mehreren Sensoren aufgezeichnet werden, um die Landezone der medizinischen Vorrichtung (501, 601) zu schätzen; und
den Strom von Bildern mit einer visuellen Anzeige (503, 603) zu versehen, die die geschätzte Landezone anzeigt;
wobei die ersten Sensordaten ein oder mehrere Bilder des Stroms von Bildern sind; und
wobei das eine oder die mehreren Bilder des Stroms von Bildern mithilfe von Datenarchitektur für maschinelles Lernen verarbeitet werden, die darauf trainiert ist, die Landezone der medizinischen Vorrichtung (501, 601) zu schätzen.

2. Bildverarbeitungsvorrichtung nach Anspruch 1, wobei die Verarbeitungseinheit ferner dazu konfiguriert ist, einen Teil der Trajektorie der medizinischen Vorrichtung, wenn sie aus dem Endoskop ausgefahren ist, durch Verarbeiten der ersten Sensordaten zu schätzen, und wobei die visuelle Anzeige ferner die geschätzte Trajektorie anzeigt.

3. Bildverarbeitungsvorrichtung nach Anspruch 1 oder 2, wobei die Verarbeitungseinheit ferner dazu konfiguriert ist:
eine Kennzeichnung einer anatomischen Landmarke in dem Strom von Bildern zu erhalten;
die Lage der anatomischen Landmarke in dem Strom von Bildern zu bestimmen;
zu bestimmen, ob sich die geschätzte Landezone mit der bestimmten Lage der anatomischen Landmarke überschneidet.

4. Bildverarbeitungsvorrichtung nach Anspruch 3, wobei die Verarbeitungseinheit ferner dazu konfiguriert ist:
die Kennzeichnung der anatomischen Landmarke durch Verarbeiten von einem oder mehreren Bildern des Stroms von Bildern zu erhalten.

5. Bildverarbeitungsvorrichtung nach Anspruch 4, wobei die Verarbeitungseinheit dazu konfiguriert ist, die Kennzeichnung der anatomischen Landmarke durch Verarbeiten des Stroms von Bildern mithilfe einer Datenarchitektur für maschinelles Lernen zu erhalten, die darauf trainiert ist, die anatomische Landmarke in Endoskopbildern zu kennzeichnen.

6. Bildverarbeitungsvorrichtung nach Anspruch 3, wobei die Verarbeitungseinheit ferner dazu konfiguriert ist:
die Kennzeichnung der anatomischen Landmarke durch eine Benutzereingabe zu erhalten.

7. Bildverarbeitungsvorrichtung nach einem der Ansprüche 3 bis 6, wobei die Verarbeitungseinheit ferner dazu konfiguriert ist:
den Strom von Bildern mit einer visuellen Anzeige zu versehen, die anzeigt, dass sich die geschätzte Landezone mit der bestimmten Lage der anatomischen Landmarke überschneidet.

8. Bildverarbeitungsvorrichtung nach einem der Ansprüche 1 bis 7, wobei das Endoskop ferner ein Führungselement umfasst, das dazu konfiguriert ist, die medizinische Vorrichtung in eine bestimmte Richtung zu führen, und wobei der eine oder die mehreren Sensoren ferner einen Führungselementsensor zum Erfassen der Position und/oder Ausrichtung des Führungselements umfassen, und wobei die ersten Sensordaten Daten aufweisen, die von dem Führungselementsensor aufgezeichnet wurden.

9. Anzeigeeinheit zum Anzeigen von Bildern, die durch eine Bilderfassungsvorrichtung eines Endoskops erhalten werden, wobei die Anzeigeeinheit eine Bildverarbeitungsvorrichtung nach einem der Ansprüche 1 bis 8 umfasst.

10. Endoskopsystem, umfassend ein Endoskop und eine Bildverarbeitungsvorrichtung nach einem der Ansprüche 1 bis 8, wobei das Endoskop eine Bildaufnahmevorrichtung aufweist und die Verarbeitungseinheit der Bildverarbeitungsvorrichtung mit der Bildaufnahmevorrichtung des Endoskops funktionell verbunden werden kann.

11. Endoskopsystem nach Anspruch 10, wobei das Endoskop ein Führungselement aufweist, das dazu konfiguriert ist, die medizinische Vorrichtung in eine bestimmte Richtung zu führen und wobei das Endoskop ein Duodenoskop ist und wobei das Führungselement ein Elevatorelement ist, das dazu konfiguriert ist, einen Katheter anzuheben.

12. Verfahren zum Trainieren einer Datenarchitektur für maschinelles Lernen zum Schätzen einer Landezone einer medizinischen Vorrichtung (501, 601), die aus einer Spitze eines Endoskops (302) ausfahrbar ist, wobei die Landezone die Position ist, die die medizinische Vorrichtung (501, 601) erreichen wird, wenn eine vorbestimmte Länge aus dem Endoskop (302) ausgefahren ist, umfassend die Schritte:
Bereitstellen einen Trainingsdatensatzes, der eine Vielzahl von Bildern umfasst, die mit einer Bildaufnahmevorrichtung (303) eines Endoskops (302) aufgenommen wurden,
Bereitstellen der Position der resultierenden Landezone der medizinischen Vorrichtung (501, 601) für jedes Bild.

13. Verfahren nach Anspruch 12, wobei das Endoskop ein Führungselement zum Führen der medizinischen Vorrichtung in eine bestimmte Richtung umfasst, wobei das Führungselement aus einer ersten Position in eine zweite Position beweglich ist, und wobei das Führungselement in verschiedenen Positionen in der Vielzahl von Bildern angeordnet ist.

## Revendications

1. Dispositif de traitement d'images (304) pour estimer une zone de réception d'un dispositif médical (501, 601) pouvant être étendu à partir d'une extrémité d'un endoscope (302), la zone de réception étant la position que le dispositif médical atteindra lorsqu'il sera étendu sur une longueur prédéterminée à partir de l'endoscope (302), l'endoscope (302) comprenant un ou plusieurs capteurs incluant un dispositif de capture d'images (303), le dispositif de traitement d'images (304) comprenant une unité de traitement (307) pouvant être connectée de manière opérationnelle au ou aux capteurs de l'endoscope (302), l'unité de traitement (307) étant configurée pour :
obtenir un flux d'images capturées par le dispositif de capture d'images (303) de l'endoscope (302) ;
traiter des premières données de capteur enregistrées par le ou les capteurs pour estimer la zone de réception du dispositif médical (501, 601) ; et
fournir au flux d'images une indication visuelle (503, 603), indiquant la zone de réception estimée ;
les premières données de capteur étant une ou plusieurs images du flux d'images ; et
la ou les images du flux d'images étant traitées à l'aide d'une architecture de données d'apprentissage automatique entraînée à estimer la zone de réception du dispositif médical (501, 601).

2. Dispositif de traitement d'images selon la revendication 1, l'unité de traitement étant en outre configurée pour estimer une partie de la trajectoire du dispositif médical lorsqu'il s'étend depuis l'endoscope, en traitant les premières données de capteur, et l'indication visuelle indiquant en outre la trajectoire estimée.

3. Dispositif de traitement d'images selon la revendication 1 ou 2, l'unité de traitement étant en outre configurée pour :
obtenir l'identification d'un repère anatomique dans le flux d'images ;
déterminer l'emplacement du repère anatomique dans le flux d'images ;
déterminer si la zone de réception estimée croise l'emplacement déterminé du repère anatomique.

4. Dispositif de traitement d'images selon la revendication 3, l'unité de traitement étant en outre configurée pour :
obtenir l'identification du repère anatomique en traitant une ou plusieurs images du flux d'images.

5. Dispositif de traitement d'images selon la revendication 4, l'unité de traitement étant configurée pour obtenir l'identification du repère anatomique en traitant le flux d'images à l'aide d'une architecture de données d'apprentissage automatique entraînée à identifier le repère anatomique dans des images d'endoscope.

6. Dispositif de traitement d'images selon la revendication 3, l'unité de traitement étant en outre configurée pour :
obtenir l'identification du repère anatomique par l'intermédiaire d'une entrée utilisateur.

7. Dispositif de traitement d'images selon l'une quelconque des revendications 3 à 6, l'unité de traitement étant en outre configurée pour :
fournir au flux d'images une indication visuelle, indiquant que la zone de réception estimée croise l'emplacement déterminé du repère anatomique.

8. Dispositif de traitement d'images selon l'une quelconque des revendications 1 à 7, l'endoscope comprenant en outre un élément de guidage configuré pour guider le dispositif médical dans une direction particulière, et le ou les capteurs comprenant en outre un capteur d'élément de guidage pour détecter la position et / ou l'orientation de l'élément de guidage, et les premières données de capteur comprenant des données enregistrées par le capteur d'élément de guidage.

9. Unité d'affichage pour afficher des images obtenues par un dispositif de capture d'images d'un endoscope, l'unité d'affichage comprenant un dispositif de traitement d'images selon l'une quelconque des revendications 1 à 8.

10. Système d'endoscope comprenant un endoscope et un dispositif de traitement d'images selon l'une quelconque des revendications 1 à 8, l'endoscope ayant un dispositif de capture d'images et l'unité de traitement du dispositif de traitement d'images pouvant être connectée de manière opérationnelle au dispositif de capture d'images de l'endoscope.

11. Système d'endoscope selon la revendication 10, l'endoscope ayant un élément de guidage configuré pour guider le dispositif médical dans une direction particulière et l'endoscope étant un duodénoscope et l'élément de guidage étant un élément d'élévation configuré pour élever un cathéter.

12. Procédé d'apprentissage d'une architecture de données d'apprentissage automatique pour estimer une zone de réception d'un dispositif médical (501, 601) extensible à partir d'une pointe d'un endoscope (302), la zone de réception étant la position que le dispositif médical (501, 601) atteindra lorsqu'il sera étendu sur une longueur prédéterminée à partir de l'endoscope (302), comprenant les étapes de :
fourniture d'un ensemble de données d'apprentissage comprenant une pluralité d'images capturées par un dispositif de capture d'images (303) d'un endoscope (302),
fourniture pour chaque image de la position de la zone de réception résultante du dispositif médical (501, 601).

13. Procédé selon la revendication 12, l'endoscope comprenant un élément de guidage pour guider le dispositif médical dans une direction particulière, l'élément de guidage étant mobile d'une première position à une seconde position, et l'élément de guidage étant agencé dans différentes positions dans la pluralité d'images.
